# EUROPEAN PATENT SPECIFICATION

(11) **EP 0 560 270 B1**
(45) Date of publication and mention of the grant of the patent: **10.07.1996**
(21) Application number: 93103714.7
(22) Date of filing: 09.03.1993
(51) Int. Cl.: A61M 5/142, F04B 43/08

(54) **Linear peristaltic pump**
Lineare Peristaltikpumpe
Pompe peristaltique linéaire

(30) Priority: 10.03.1992 GR 92010089
(43) Date of publication of application: 15.09.1993
(73) Proprietor: MICREL LTD, MICROELECTRONIC APPLICATIONS CENTER, GR-156 69 Athens (GR)
(72) Inventor: Tsoukalis, Alexandre, 162 33 Vironas Athens (GR)
(74) Representative: Eisenführ, Speiser & Partner

(56) References cited:
- EP-A- 0 422 855
- EP-A- 0 426 273
- WO-A-84/00690
- DE-A- 4 035 182
- GB-A- 2 020 735
- US-A- 4 909 710

## Description

The invention relates to a linear peristaltic pump comprising a flexible tube supported on a support plate and having an input end connected to a fluid reservoir as well as an output end; a motor-driven cam shaft rotatably supported in a stationary frame and arranged in parallel to the flexible tube and having at least three cams, which are displaced angularly to each other; a cam follower for each cam, guided between parallel guide faces for straight reciprocable movement in a direction normal to the axis of the cam shaft, having end faces for squeezing the flexible tube in an operation sequence travelling along the tube from the input end to the output end; in which the cams, cam followers and ball bearings are substantially in the same plane.

The invention particularly relates to a portable linear peristaltic pump operated on batteries for delivering drugs to patients.

In modern therapy there is an increasing tendency of infusing drugs in home treatment in order to save expensive beds in hospitals. Linear peristaltic pumps are an improvement over the older type of rotary peristaltic pumps, and in their portable form, linear peristaltic pumps have the advantage that disposable parts of the mechanism are less expensive to replace than in reciprocally moving piston pumps in which the pumping mechanism is installed on the disposable parts like syringes.

In linear peristaltic pumps, the drug is contained in a reservoir of soft material connected to a small flexible silicone tube which is consecutively compressed along its length by a set of fingers driven by a cam mechanism, thereby pumping the drug from the reservoir into a catheter. Large size bedside volumetric linear peristaltic pumps as used in hospitals are reliable long-life devices which are suitable for intensive care units.

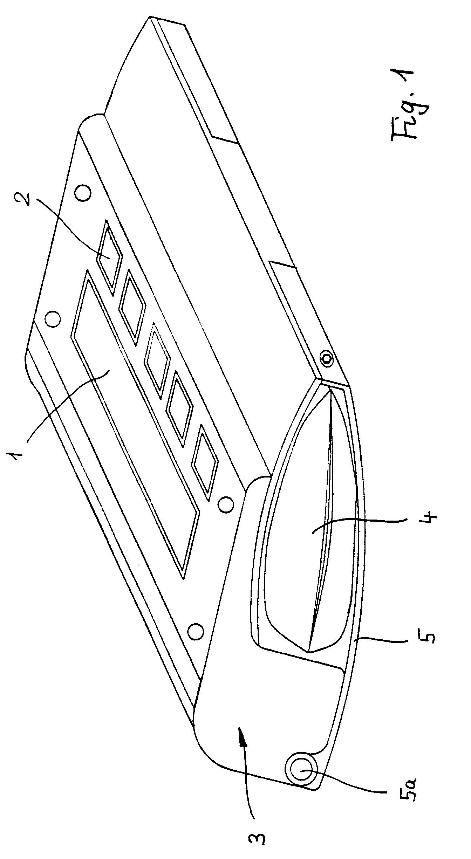

EP-A-214 443 shows a transfusion pump having a flexible tube supported on a support plate and having an input end connected to a fluid reservoir, and an output end connected, for instance, to a catheter. The mechanism comprises a motor-driven cam shaft arranged in parallel to the flexible tube and having a plurality of cams. In a first embodiment (Figure 1) these cams directly operate on cam fingers which squeeze the flexible tube in an operation sequence travelling along the tube from its input end to the output end. It is clear that between the cams and the cam followers large friction exists which would be a disadvantage in case of portable pumps operating on batteries.

EP-A-214 443 also discloses a pump mechanism (Figs. 7 and 8) which uses ball bearings which are fixed excentrically on a shaft, and the outer circumferences of these ball bearings directly operate on the flexible tube. Although in this construction friction and wear is reduced, rather large ball bearings are necessary in order to obtain sufficiently large strokes for squeezing the flexible tube. Such large ball bearings need larger space in a direction normal to the squeezing direction of the flexible tube, so that this principle is more suitable for large bedside pumps than for portable pumps. Moreover, excentric pump mechanisms have the disadvantage that the operation curves can only be made sinusodial but not in any shape as would be possible with shaped cam surfaces.

It is therefore an object of the present invention to provide a linear peristaltic pump of small size and low friction and wear of the mechanism, and consequently lower power consumption, the reliability of which is about the same as that of larger bedside volumetric pumps.

According to the invention, a linear peristaltic pump as defined above is characterized in that it further comprises ball bearings for reducing friction between the cams and the cam followers; that the inner parts of the ball bearings are supported on the cam followers whereas the cams operate on the outer circumferential surfaces of the ball bearings; and that the overall diameter of the ball bearings is smaller than that of the outer surfaces of the cams.

Although it is already known to use cam followers in peristaltic pumps having rollers operating on cam surfaces (EP-A-446 898 and GB-A-2 020 735), the pumps described in these publications are of such a construction that they do not permit small sizes necessary for portable pumps operated on batteries. The mechanism according to EP-A-446 898 uses three cam followers placed at different angular positions of one cam in order to squeeze the flexible tube at only three positions, the whole mechanism needing rather large space in a direction normal to the direction of movement. The mechanism of GB-A-2 020 735 has a cam follower supported on a sufficiently long support arm, so that the finger operating on the flexible tube moves substantially on a straight line. The long support arm does not permit a small width of the mechanism, either. Accordingly, these two constructions are not suitable for a linear peristaltic pump of small dimensions.

In case of the peristaltic pump according to the invention, the straight reciprocable movement of the cam followers is guided between parallel guide faces, and the dimensions normal to the direction of movement can be kept small in spite of the use of ball bearings reducing friction on the cams, because smaller ball bearings can be used compared with ball bearings mounted excentrically on a shaft in the prior art pumps.

In a preferable embodiment, a stationary frame is provided with parallel side walls with guide grooves for guiding side faces of the cam followers. The ball bearings are preferably supported on projections extending parallel to the axis of the cam shaft from the cam followers.

In order to avoid movements of the flexible tube in transversal direction normal to the operating direction of the cam followers, the flexible tube is preferably held in position under the cam followers by stationary shoulders limiting sideward movement, the height of the shoulders being less than twice the wall thickness of the tube. To avoid the entering of fluid into the pump mechanism in case the flexible tube is defective, preferably a protective sheet is placed between the flexible tube and the end faces of the cam followers for sealing the drive mechanism against the tube.

In order to provide rigidity of the pump mechanism and maintain exact pumping volumes, the cam shaft with its cams is preferably a one-piece metal part, preferably made by powder injection molding or powder metal sintering.

In a linear peristaltic pump it is important that the pumping volume is exact and even, substantially independent from tolerances and mechanical properties of the flexible tube. In order to obtain such characteristics, in a preferred embodiment, each cam has three curve portions:
- a first portion having a continuously increasing radius for the pressing phase,
- a second portion having a constant radius equal to the radius at the end of the first portion for maintaining the flexible tube closed, and
- a third portion having a sharp decrease of the radius for quick release of the tube.

With such a curvature of the cams the flexible tube is squeezed over a rather long angle of rotation, and released and expanded over a rather short angle of rotation, so that the tube has sufficient expansion time to reach its full and expanded form to allow its filling with fluid to the full volume.

In order to provide an even torque load to the driving motor, the cams are displaced at even angles around a full circle. The second portion of the curvature is made slightly longer than said angle to provide overlapping of the second portions of the individual cams, so that the volume of fluid to be pumped is safely trapped between the closed sections of the tube without any back flow. In case of six regular cams and angular displacements between the cams of 60°, the duration of the first portion is about 200°, the duration of the second portion about 65° and the duration of the third portion about 95° of a full revolution of 360°.

In order to make the fluid flow to the output even without any large variations, preferably an additional cam and cam follower are provided at the output end of the flexible tube for linearizing the output flow, the maximum radius of this cam being less than necessary to close the tube. This additional cam has the function to increase the output flow for the short period of time when the last regular cam is in its closed position, i.e. the additional cam has a first portion of depression during the second portion of the last regular cam, and a second portion of release of pressure to the flexible tube during the remaining part of the revolution. In case of the above example with six regular cams, the duration of the first portion of the additional cam is about 65° and the duration of the second portion is about 295°.

Another embodiment, which is however not covered by claim 1 is a special construction of placing the fluid reservoir and the flexible tube within a compartment of a housing of the peristaltic pump. This embodiment is characterized by an exchangeable unit comprising the fluid reservoir, preferably made from soft elastomer material, and the flexible tube having its input end connected to the fluid reservoir. The exchangeable unit is placed in a compartment of the housing which is closed by a lid constituting the support plate to the flexible tube. By this construction, the fluid reservoir together with the flexible tube can be replaced as disposable parts, so that the pumping mechanism can be used for pumping different fluids, or for different patients.

In order to make the peristaltic pump having such exchangeable units safe, preferably registration means are provided at a frame of the exchangeable unit and within the compartment, which registration means only permit insertion of the exchangeable unit in proper orientation but not in reverse orientation.

In order to avoid leakage of the fluid contained in the reservoir or in the flexible tube during exchange of the exchangeable unit, preferably clamping means are provided at the output end of the flexible tube, the clamping means opening the output if the exchangeable unit is within the compartment and the lid is properly closed. The clamping means are preferably spring-biased into the closed position and opened by a projection of the lid.

In order to increase the safety of the linear peristaltic pump according to the invention, a pressure sensor for sensing the output pressure of the peristaltic pump is provided. The pressure sensor, in a preferred embodiment, is operated by a flexible, soft membrane covering an opening of a housing connected to the output end of the flexible tube. The soft membrane has a rather large area, so that the overall sensitivity of the pressure sensor is increased.

The invention is now described in connection with preferable embodiments of the invention in connection with the accompanying drawings.
- Figure 1: shows a perspective view of the linear peristaltic pump according to the invention;
- Figure 2: shows a cross section through the pumping mechanism, showing the cam shaft, cam followers with ball bearings and the flexible tube;
- Figure 3: shows a side view of the pumping mechanism of the peristaltic pump;
- Figure 4: shows a perspective view of an exchangeable unit comprising the fluid reservoir and the flexible tube;
- Figure 5: shows a side view of one of the regular cams; and
- Figure 6: shows a side view of an additional cam for equalizing the output fluid volume.

The general view of the linear peristaltic pump, Figure 1, shows a housing 3 containing the mechanical parts of the pump including a motor, a reduction gear, an electric battery and the movement mechanism in the area of the rear part, whereas in a lower front part of the housing the electronics are installed using a microprocessor and SMD technology for reducing size. The housing 3 is provided with a lid 5 at the bottom hinged at 5a for access to a compartment into which an exchangeable unit comprising a fluid reservoir 4 can be inserted. At the top of the housing, a display 1 and operation keys 2 are provided.

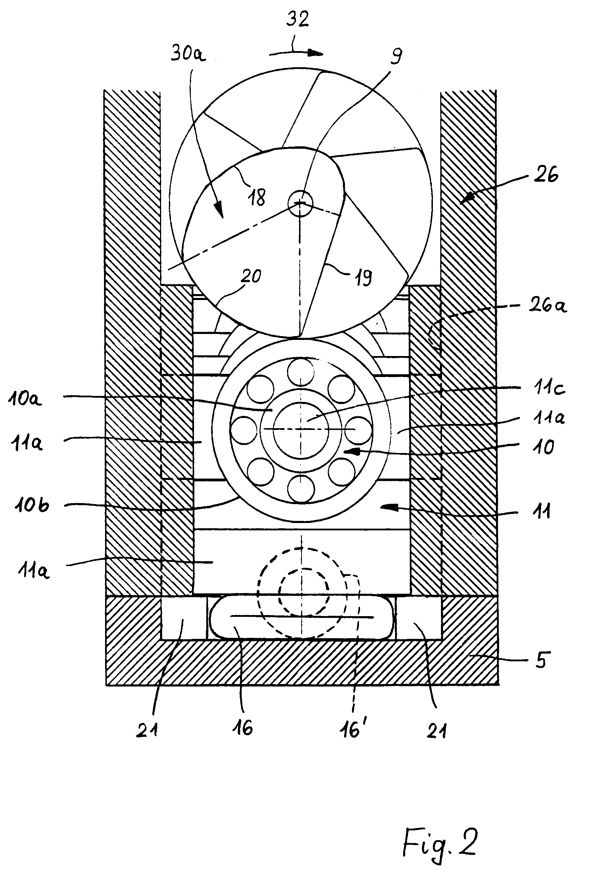

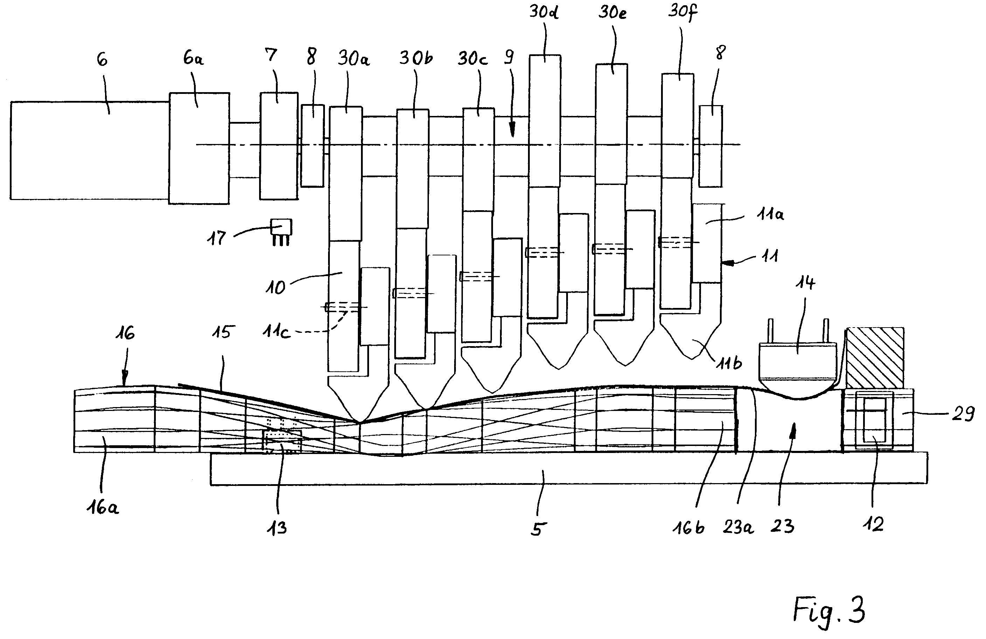

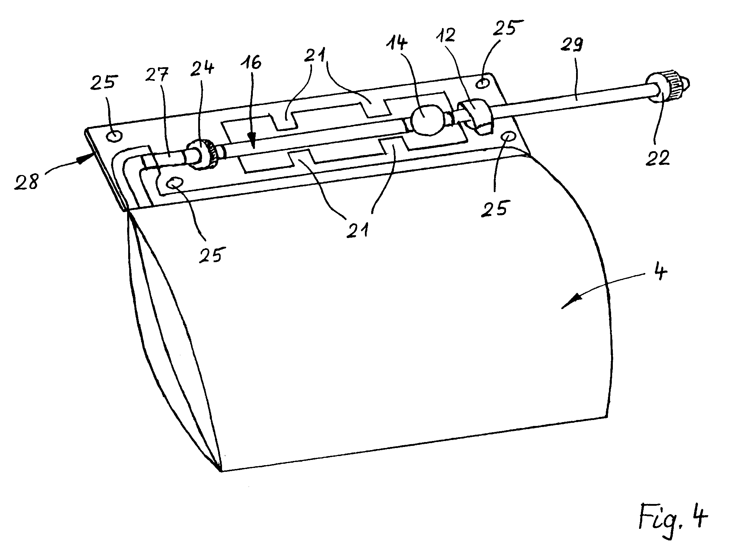

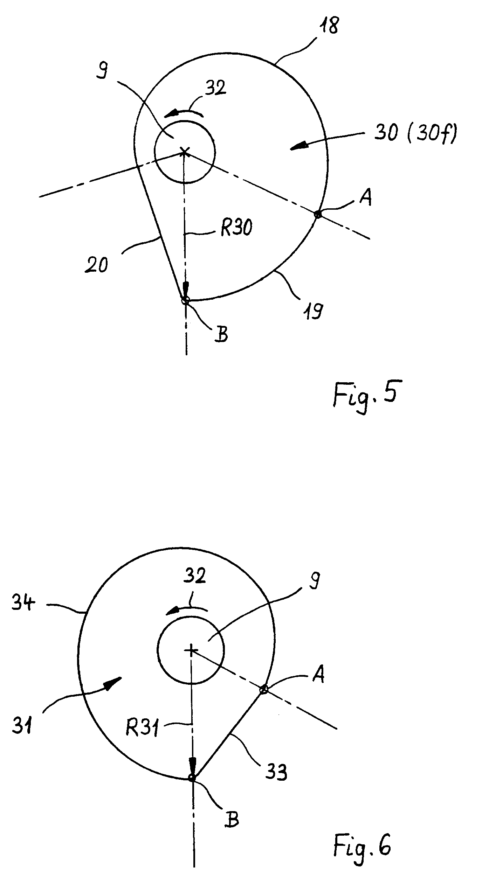

Now, the pumping mechanism shall be described in connection with Figures 2 and 3 of the drawings. A cam shaft 9 having a plurality of cams 30a-30f is rotatably supported by journals 8 within a stationary frame 26. The cam shaft 9 is driven by a low inertia motor 6 having a reduction gear 6a. A magnetic drum 7 having angularly spaced magnets is operating on hall sensors 17 which are angularly displaced to each other in order to detect the direction of rotation as well as the speed of rotation. The signals of the hall sensors 17 are used in a microprocessor controlling the operation of the peristaltic pump.

Corresponding to each of the cams 30a-30f, there is provided a plurality of cam followers 11 each having a small ball bearing 10 supported with is inner part 10a on a projection 11c formed at the cam followers 11. The outer circumference 10b of each ball bearing 10 touches the cam surface of each cam 30a-30f, so that only small friction is present between the cam followers 11 and the cams 30a-30f.

End faces 11b of the cam followers 11 operate on a flexible tube 16 supported on a support plate 5, being the lid for the housing 3 as already described. The cam followers 11 are guided for movement in a straight line in vertical direction (as seen in the drawings) by side faces 11a moving in guide faces 26a in the form of grooves present in side walls of the stationary frame 26.

From Figures 2 and 3 it can be seen that the end face 11b of the first cam 30a has fully squeezed the flexible tube 16 to its closed position (the dotted lines showing the flexible tube 16' in its fully open position). The individual cams 30a-30f have all the same shape but are displaced angularly to each other by 60°. This displacement provides for a substantially even torque load to the motor. In Figure 3, the input end 16a of the flexible tube 16 is seen on the left side of the drawing, whereas the output end 16b is shown on the right side.

The flexible tube 16 is made of elastic material, preferably of silicone material. To seal the flexible tube 16 against the drive mechanism, a protective sheet 15 of plastic material is placed between the tube 16 and the end faces 11b of the cam followers 11 (only shown in Figure 3 but not in Figure 2 for the sake of clarity).

The cam 30a as shown in Figure 2 has three portions of curvature, i.e.
- a first portion 18 having a continuously increasing radius of calculated curvature for the pressure phase,
- a second portion 19 having a constant radius equal to the radius at the end of the first portion 18 for maintaining the flexible tube 16 fully closed, and
- a third portion 20 having a sharp decrease of the radius for quick release of the tube 16.

From Figure 2 it can be seen that, although the stroke of the cams 30 can be rather large, the width of the mechanism (seen in horizontal direction of Figure 2) does not need to be much larger than the maximum outer diameter (portion 20) of the cams. The bearings 10 which can be small, only take space in the squeezing direction, and the sliding support of the cam followers 11 within the stationary support 26 safeguards a linear guidance in the squeezing direction.

The cam followers 11 are pushed upwards by the elasticity of the tube 16 upon release by the cam followers 11. It is, however, also possible to assist the upward movement by return springs.

Figure 4 now shows an exchangeable unit comprising the fluid reservoir 4 and the flexible tube 16 supported on a frame 28 made of hard plastic material and connected to the fluid reservoir. The fluid reservoir 4 is in the form of a small bag preferably made from soft elastomer material, and the flexible tube 16 is connected through a pump connector 24 and an input tube 27 to the fluid reservoir. The frame 28 comprises registration means 25 in the form of holes, into which complementary projections within the compartment of the housing 3 would fit in order to safeguard proper orientation of the exchangeable unit within the compartment. The output end 16b of the flexible tube 16 is connected to a small housing 23 which is only shown schematically in Figure 3. An opening at the upper side (as seen in the drawing) is closed by a membrane 23a glued to the walls of the housing at the opening. The output pressure at the output end 16b of the flexible tube 16 is present in the housing 23 and operates the membrane 23a having a rather large area for operating a pressure sensor 14 which can be an analog pressure sensor or a switch. The pressure sensor 14 is used for sensing overpressure at the output, so that either the operation of the peristaltic pump can be stopped and/or an alarm signal be given. The housing 23 is connected to an output tube 29, and from there to an output connector 22, in the present case a Luer Lock connector (particularly see Figure 4).

The frame 28 also has projections 21 which safeguard that the flexible tube 16 does not move transversely under the squeezing pressure of the cam followers 11. These projections or shoulders 21 are also shown in Figure 2. Their height in squeezing direction is slightly less than twice the wall thickness of the tube 16, so that the tube can be fully compressed by the end faces 11b of the cam followers 11.

As already described in connection with Figure 1, the exchangeable unit comprising the fluid reservoir 4 and the flexible tube 16, can be inserted into a compartment of the housing 3 by opening the hinged lid 5 at the bottom of the housing 1. In order that no fluid can leak out from the fluid reservoir 4 or the flexible tube 16 during transport and exchange, a spring clamp 12 is provided at the output tube (29). This spring clamp 12 is spring-biased into its closed position. When the exchangeable unit is placed into the compartment of the housing 3 and the lid 5 is properly closed, a projection (not shown) at the lid 5 operates the spring clamp 12 into its open position, so that the output is opened and the peristaltic pump can be used.

Moreover, as can be seen in Figure 3, a switch 13 is provided which is operated by the lid 5 in such a way that operation of the peristaltic pump is only permitted when the lid 5 is properly closed.

Now, more details of the shape of the regular six cams 30a-30f shall be described in connection with Figure 5. As already mentioned in connection with Figures 2 and 3, each of the six cams 30a-30f has a first portion 18 having a continuously increasing radius of calculated curvature, and in the present embodiment, this portion extends over an angle of 200°. The radius of the cam surface preferably increases linearly with the rotation angle in the direction of an arrow 32. The subsequent second portion 19 has a constant radius, i.e. the maximum radius R30. In the present embodiment, the constant radius in portion 19 lasts for 65°. In the third portion 20 a sharp decrease from the maximum radius R30 to the minimum radius takes place, in the present embodiment during a rotation angle of about 95°. The maximum radius R30 and the length of the cam followers 11 are such that the flexible tube 16 is completely closed. Although in Figure 3, the end faces 11b of some of the cam followers 11 are shown at a distance from the flexible tube 16, this distance or gap is smaller or zero in practice.

Figure 6 now shows an additional cam 31 which is placed behind the sixth regular cam 30f (Figure 3) for the purpose of smoothing variations of the output pressure during the pumping action. This additional cam is not shown in Figure 3 but only in Figure 6.

As Figure 6 shows, the additional cam 31 has a first portion 33 during which the radius increases from a minimum value to a maximum radius R31. From this maximum value R31 the radius decreases during a second portion 34 to the minimum value. The maximum radius R31 is smaller than the maximum radius R30 of the regular cams 30 because the flexible tube 16 shall only be pressed but not closed. The angular duration of the first portion 33 is about 65°, whereas the remaining portion 34 is about 295°.

Figure 5 now shows the angular position of the last and sixth regular cam 30f, and the additional cam 31 is fixed to the cam shaft 9 at the same angular position as shown for the sixth cam 30f in Figure 5, i.e. when cam 30f has its maximum radius R30, the additional cam 31 also has it maximum radius R31. From this follows that when the sixth cam 30f rotates from point A to point B during its second portion 19 during which the flexible tube 16 is held fully closed, the additional cam 31 runs from point A to point B through its first portion 33 during which the pressure to the end portion 16b of the flexible tube 16 is gradually increased without closing the tube 16. This means that while no fluid is pumped beyond the closed cam 30f the additional cam 31 provides a pumping action to the output during the same time. Accordingly, pressure variations at the output are greatly reduced.

## Claims

1. Linear peristaltic pump comprising
a flexible tube (16) supported on a support plate (5) and having an input end (16a) connected to a fluid reservoir (4) as well as an output end (16b);
a motor-driven cam shaft (9) rotatably supported in a stationary frame (26) and arranged in parallel to the flexible tube (16) and having at least three cams (30a-30f), which are displaced angularly to each other;
a cam follower (11) for each cam, guided between parallel guide faces (26a) for straight reciprocating movement in a direction normal to the axis of the cam shaft (9), having end faces (11b) for squeezing the flexible tube (16) in an operation sequence travelling along the tube from the input end (16a) to the output end (16b); in which the cams (30), the cam followers (11) and ball bearings (10) are substantially in the same plane;
characterized in that it further comprises ball bearings for reducing friction between the cams (30) and the cam followers (11); that
the inner parts (10a) of the ball bearings (10) are supported on the cam followers (11) whereas the cams (30) operate on the outer circumferential surfaces (10b) of the ball bearings (10); and
that the overall diameter of the ball bearings (10) is smaller than that of the outer surfaces (19) of the cams (30).

2. Linear peristaltic pump according to claim 1, characterized by the stationary frame (26) having parallel side walls with guide grooves (26a) for guiding side faces (11a) of the cam followers (11).

3. Linear peristaltic pump according to claim 1 or 2, characterized in that the ball bearings (10) are supported on projections (11c) extending parallel to the axis of the cam shaft (9) from the cam followers (11).

4. Linear peristaltic pump according to any preceding claim, characterized in that the flexible tube (16) is held in position under the cam followers (11) by stationary shoulders (21) limiting sideward movement, the height of the shoulders (21) being less than twice the wall thickness of the tube (16).

5. Linear peristaltic pump according to any preceding claim, characterized by a protective sheet (15) between the flexible tube (16) and the end faces (11b) of the cam followers (11) for sealing the drive mechanism against the tube (16).

6. Linear peristaltic pump according to any preceding claim, characterized in that the cam shaft (9) with its cams (30a-30f) is a one-piece metal part, preferably made by powder injection molding or powder metal sintering.

7. Linear peristaltic pump according to any preceding claim, characterized in that each cam (30) has three curve portions:
- a first portion (18) having a continuously increasing radius for the pressing phase,
- a second portion (19) having a constant radius equal to the radius at the end of the first portion (18) for maintaining the flexible tube (16) closed, and
- a third portion (20) having a sharp decrease of the radius for quick release of the tube (16).

8. Linear peristaltic pump according to claim 7, characterized in that the cams (30a-30f) are displaced at even angles around a full circle, and that the second portion (19) is slightly longer than said angle to provide overlapping of the second portions (19) of the individual cams.

9. Linear peristaltic pump according to claim 7 or 8, characterized in that in case of six regular cams (30a-30f) and angular displacements between the cams of 60°,
- the duration of the first portion (18) is about 200°,
- the duration of the second portion (19) is about 65°, and
- the duration of the third portion (20) is about 95° of a full revolution of 360°.

10. Linear peristaltic pump according to anyone of claims 7 to 9, characterized by an additional cam (31) and cam follower at the output end (16b) of the flexible tube (16) for linearizing the output flow, the maximum radius of this cam (31) being less than necessary to close the tube (16).

11. Linear peristaltic pump according to claim 10, characterized in that the additional cam (31) has a first portion (33) for generation of pressure during the second portion (19) of the last regular cam (30f), and a second portion (34) for release of pressure to the flexible tube (16).

12. Linear peristaltic pump according to claims 9 and 11, characterized in that the duration of the first portion (33) of the additional cam (31) is about 65° and the duration of the second portion (34) is about 295°.

13. Linear peristaltic pump according to any preceding claim, characterized by a pressure sensor (14) for sensing the output pressure of the peristaltic pump, the pressure sensor (14) being operated by a flexible, soft membrane (23a) covering an opening of a housing (23) connected to the output end (16b) of the flexible tube (16).

## Patentansprüche

1. Lineare Schlauchpumpe mit
einem auf einer Halterungsplatte (5) gehalterten flexiblen Schlauch (16) mit einem Eingabeende (16a), das mit einem Fluidbehälter (4) verbunden ist, sowie mit einem Ausgabeende (16b);
mit einer motorgetriebenen Nockenwelle (9), die drehbar in einem stationären Rahmen (26) gehaltert und parallel zum flexiblem Schlauch (16) angeordnet ist und mindestens drei Nocken (30a - 30f) aufweist, die im Winkel zueinander versetzt sind;
mit einem Stößel (11) für jede Nocke, der zwischen parallel verlaufenden Führungsflächen (26a) zur geraden Hin- und Herbewegung in zur Achse der Nockenwelle (9) senkrecht verlaufenden Richtung geführt wird und Endflächen (11b) zum Zusammendrücken des flexiblen Schlauches (16) in einer Betätigungssequenz aufweist, die vom Eingabeende (16a) den Schlauch entlang zum Ausgabeende (16b) verläuft, wobei die Nocken (30), die Stößel (11) und Kugellager (10) im wesentlichen in der gleichen Ebene angeordnet sind,
dadurch gekennzeichnet,
daß die Kugellager zur Reibungsverringerung zwischen Nocken (30) und Stößel (11) vorgesehen sind,
daß die inneren Teile (10a) der Kugellager (10) auf den Stößeln (11) gelagert sind, wohingegen die Nocken (30) auf die äußeren Umfangflächen (10b) der Kugellager (10) wirken, und
daß der Gesamtdurchmesser der Kugellager (10) kleiner ist als der der Außenflächen (19) der Nocken (30).

2. Lineare Schlauchpumpe nach Anspruch 1,
dadurch gekennzeichnet, daß der stationäre Rahmen (26) parallel angeordnete Seitenwände mit Führungsnuten (26a) zum Führen von Seitenflächen (11a) der Stößel (11) aufweist.

3. Lineare Schlauchpumpe nach Anspruch 1 oder 2,
dadurch gekennzeichnet, daß die Kugellager (10) auf Vorsprüngen (11c) gelagert sind, die sich parallel zur Achse der Nockenwelle (9) von den Stößeln (11) aus erstrecken.

4. Lineare Schlauchpumpe nach einem der vorhergehenden Ansprüche,
dadurch gekennzeichnet, daß der flexible Schlauch (16) von stationären Schultern (21), die die seitliche Bewegung begrenzen, in seiner Position unter den Stößeln (11) gehalten wird, wobei die Höhe der Schultern (21) geringer ist als das Zweifache der Wandstärke des Schlauches (16).

5. Lineare Schlauchpumpe nach einem der vorhergehenden Ansprüche, gekennzeichnet durch eine Schutzfolie (15) zwischen dem flexiblen Schlauch (16) und den Endflächen (11b) der Stößel (11), um den Antriebsmechanismus gegen den Schlauch (16) abzudichten.

6. Lineare Schlauchpumpe nach einem der vorhergehenden Ansprüche, dadurch gekennzeichnet, daß die Nockenwelle (9) mit ihren Nocken (30a - 30f) ein einteiliger Metallgegenstand ist, der vorzugsweise im Pulverspritzguß oder durch Pulvermetallsinterung gefertigt wurde.

7. Lineare Schlauchpumpe nach einem der vorhergehenden Ansprüche, dadurch gekennzeichnet, daß jede Nocke (30) drei Bogenabschnitte (30) aufweist:
- einen ersten Abschnitt (18) mit fortlaufend zunehmendem Radius für die Druckphase,
- einen zweiten Abschnitt (19) mit konstantem Radius, der gleich dem Radius am Ende des ersten Abschnittes (18) ist, um den flexiblen Schlauch (16) in verschlossenem Zustand zu halten, und
- einen dritten Abschnitt (20), der zur schnellen Freigabe des Schlauches (16) eine steil verlaufende Verringerung des Radius aufweist.

8. Lineare Schlauchpumpe nach Anspruch 7,
dadurch gekennzeichnet, daß die Nocken (30a - 30f) bei gleichen Winkeln in einem vollen Kreis gegeneinander versetzt sind und daß der zweite Abschnitt (19) geringfügig länger ist als der genannte Winkel, um ein Überlappen der zweiten Abschnitte (19) der einzelnen Nocken zu erzeugen.

9. Lineare Schlauchpumpe nach Anspruch 7 oder 8,
dadurch gekennzeichnet, daß bei sechs regulären Nocken (30a - 30f) und einem Versatzwinkel von 60° zwischen den Nocken
- die Dauer des ersten Abschnittes (18) etwa 200°,
- die Dauer des zweiten Abschnittes (19) etwa 65°
- und die Dauer des dritten Abschnittes (20) etwa 95° einer vollen Umdrehung von 360° beträgt.

10. Lineare Schlauchpumpe nach einem der Ansprüche 7 bis 9,
gekennzeichnet durch eine zusätzliche Nocke (31) und einen zusätzlichen Stößel am Ausgabeende (16b) des flexiblen Schlauchs (16) zum Linearisieren des Ausgabeflusses, wobei der größte Radius dieser Nocke (31) kleiner ist, als notwendig wäre, um den Schlauch (16) zu verschließen.

11. Lineare Schlauchpumpe nach Anspruch 10,
dadurch gekennzeichnet, daß die zusätzliche Nocke (31) einen ersten Abschnitt (33) zur Druckerzeugung während des zweiten Abschnittes (19) der letzten regulären Nocke (30f) und einen zweiten Abschnitt (34) zur Verminderung des Druckes auf den flexiblen Schlauch (16) aufweist.

12. Lineare Schlauchpumpe nach den Ansprüchen 9 und 11,
dadurch gekennzeichnet, daß die Dauer des ersten Abschnittes (33) der zusätzlichen Nocke (31) etwa 65° und die Dauer des zweiten Abschnittes (34) etwa 295° beträgt.

13. Lineare Schlauchpumpe nach einem der vorhergehenden Ansprüche, gekennzeichnet durch einen Drucksensor (14) zum Messen des Ausgabedrucks der Schlauchpumpe, wobei der Drucksensor (14) durch eine flexible, weiche Membrane (23a) betätigt wird, die die Öffnung eines Gehäuses (23) bedeckt, das mit dem Ausgabeende (16b) des flexiblen Schlauches (16) verbunden ist.

## Revendications

1. Pompe péristaltique linéaire comprenant un tube flexible (16) supporté sur une plaque support (5) et possédant une extrémité d'entrée (16a) reliée à un réservoir de fluide (4) ainsi qu'une extrémité de sortie (16b) ; un arbre à cames (9) actionné par un moteur, supporté de façon rotative dans un cadre fixe (26) et disposé de façon parallèle au tube flexible (16) et possédant au moins trois cames (30a-30f), qui sont décalées de façon angulaire l'une par rapport à l'autre ; une contre-came (11) pour chaque came, guidée entre deux faces de guidage parallèles (26a) pour effectuer un mouvement alternatif rectiligne dans une direction perpendiculaire à l'axe de l'arbre à cames (9), possédant des surfaces terminales (11b) pour presser le tube flexible (16) au cours d'une opération de déplacement séquentiel le long du tube, depuis l'extrémité d'entrée (16a) jusqu'à l'extrémité de sortie (16b) ; les cames (30), les contre-cames (11) et les roulements à billes (10) étant sensiblement dans le même plan ;
caractérisée en ce qu'elle comprend en outre des roulements à billes pour
réduire la friction entre les cames (30) et les contre-cames (11) ; en ce que les parties intérieures (10a) des roulements à billes (10) sont supportées sur les contre-cames (11) tandis que les cames (30) fonctionnent sur les surfaces circonferentielles extérieures (10b) des roulements à billes (10) ; et que le diamêtre hors-tout des roulements à billes (10) est inférieur à celui des surfaces extérieures (19) des cames (30).

2. Pompe péristaltique linéaire selon la revendication 1, caractérisée en ce que le cadre fixe (26) possède des parois latérales parallèles munies de rainures de guidage (26a) pour guider les faces latérales (11a) des contre-cames (11).

3. Pompe péristaltique linéaire selon la revendication 1ou 2, caractérisée en ce que les roulements à billes (10) sont supportés sur des prolongements (11c) des contre-cames (11), s'étendant parallèlement à l'axe de l'arbre à cames (9).

4. Pompe péristaltique linéaire selon l'une quelconque des revendications précédentes, caractérisée en ce que le tube flexible (16) est maintenu en position, sous les contre-cames (11), par des épaulements fixes (21) limitant le mouvement latéral, la hauteur des épaulements (21) étant inférieure à deux fois l'épaisseur de la paroi du tube (16).

5. Pompe péristaltique linéaire selon l'une quelconque des revendications précédentes, caractérisée par une feuille protectrice (15) placée entre le tube flexible (16) et les surfaces terminales (11b) des contre-cames (11) pour assurer l'étanchéité du mécanisme de propulsion contre le tube (16).

6. Pompe péristaltique linéaire selon l'une quelconque des revendications précédentes, caractérisée en ce que l'arbre à cames (9) forme avec ses cames (30a-30f) une partie métallique en une seule pièce, fabriquée de préférence par moulage par injection de poudre ou par frittage de poudre métallique.

7. Pompe péristaltique linéaire selon l'une quelconque des revendications précédentes, caractérisée en ce que chaque came (30) possède trois portions courbes :
- une première portion (18) présentant un rayon croissant de façon continue, pour la phase de pression,
- une seconde portion (19) présentant un rayon constant égal au rayon de l'extrémité de la première portion (18), pour maintenir fermé le tube flexible (16),et
- une troisième portion (20) présentant une rapide décroissance du rayon, pour une libération rapide du tube (16).

8. Pompe péristaltique linéaire selon la revendication 7, caractérisée en ce que les cames (30a-30f) sont décalées selon des angles réguliers autour d'un cercle complet, et en ce que la seconde portion (19) est légèrement plus longue que ledit angle pour fournir un chevauchement des secondes portions (19) des cames individuelles.

9. Pompe péristaltique linéaire selon la revendication 7 ou 8, caractérisée en ce que dans le cas de six cames normales (30a-30f) et de décalages angulaires de 60_ entre les cames,
- l'étendue de la première portion (18) est d'environ 200_,
- l'étendue de la seconde portion (19) est d'environ 65_, et
- l'étendue de la troisième portion (20) est d'environ 95_ d'une révolution totale de 360_.

10. Pompe péristaltique linéaire selon l'une quelconque des revendications 7 à 9, caractérisée par une came additionnelle (31) et une contre-came placées à l'extrémité de sortie (16b) du tube flexible (16) pour linéariser le débit de sortie, le rayon maximum de cette came (31) étant inférieur à celui nécessaire pour fermer le tube (16).

11. Pompe péristaltique linéaire selon la revendication 10, caractérisée en ce que la came additionnelle (31) possède une première portion (33) de dépression pendant la seconde portion (19) de la dernière came normale (30f), et une seconde portion (34) de libération de la pression sur le tube flexible (16).

12. Pompe péristaltique linéaire selon les revendications 9 et 11, caractérisée en ce que l'étendue de la première portion (33) de la came additionnelle (31) est d'environ 65_ et l'étendue de la seconde portion (34) est d'environ 295_.

13. Pompe péristaltique linéaire selon l'une quelconque des revendications précédentes, caractérisée par un capteur de pression (14) pour détecter la pression de sortie de la pompe péristaltique, le capteur de pression (14) étant actionné par une membrane flexible, souple (23a) couvrant une ouverture d'un logement (23) relié à l'extrémité de sortie (16b) du tube flexible (16).
